# EUROPÄISCHE PATENTSCHRIFT

(11) **EP 0 946 815 B1**
(45) Veröffentlichungstag und Bekanntmachung des Hinweises auf die Patenterteilung: **13.03.2002**
(21) Anmeldenummer: 97953658.8
(22) Anmeldetag: 19.12.1997
(51) Int. Cl.: D21B 1/04

(54) **VERFAHREN UND VORRICHTUNG ZUR PROZESSFÜHRUNG BEI DER HERSTELLUNG VON FASERSTOFF AUS HOLZ**
MEANS AND DEVICE FOR CONDUCTING A PROCESS IN THE PRODUCTION OF FIBROUS MATERIAL FROM WOOD
PROCEDE ET DISPOSITIF POUR CONDUIRE UN PROCESSUS LORS DE LA FABRICATION DE MATIERE FIBREUSE A PARTIR DE BOIS

(30) Priorität: 20.12.1996 DE 19653532
(43) Veröffentlichungstag der Anmeldung: 06.10.1999
(73) Patentinhaber: SIEMENS AKTIENGESELLSCHAFT, 80333 München (DE)
(72) Erfinder: ZEINER, Gerhard, D-70563 Stuttgart (DE); FURUMOTO, Herbert, D-91052 Erlangen (DE); LAMPE, Uwe, D-21614 Buxtehude (DE); ROTH, Christoph, D-81739 München (DE); KESSLER, Rudolf, D-72762 Reutlingen (DE)
(86) Internationale Anmeldenummer: DE9702989
(87) Internationale Veröffentlichungsnummer: WO9828486

(56) Entgegenhaltungen:
- WO-A-91/10904
- WO-A-93/15389
- DE-A- 3 712 096
- DE-A- 19 510 008

## Beschreibung

Die Erfindung bezieht sich auf ein Verfahren zur Prozeßführung und Prozeßoptimierung bei der Herstellung von Faserstoff aus Holz, insbesondere von Holzschliff und/oder Refinerstoff, durch Mahlen und/oder Schleifen des Holzes und Überführen des Holzes in eine Stoffsuspension unter Einsatz wenigstens eines Zustands- und/oder Prozeßmodells. Daneben bezieht sich die Erfindung auch auf die zugehörige Vorrichtung zur Durchführung des Verfahrens.

Einer der Ausgangsstoffe des für die Herstellung von Papier ist ein aus dem Rohstoff Holz auf mechanischem Wege gewonnener Faserstoff. Dieser wird entweder unmittelbar durch Abschleifen von Baumstämmen als sogenannter Holzschliff oder aber in einem ein- oder mehrstufigen Prozeß als Refiner-Stoff aus Hackschnitzeln hergestellt. Die Herstellung solcher Faserstoffe stellt an die Prozeßführung der Schleifer oder der Refiner, insbesondere an die Prozeßführung mit Automatisierungsgeräten, erhebliche Anforderungen.

Die Prozeßführung von Refinern zur Herstellung von Holzstoff für die Erzeugung von Papier ist dadurch gekennzeichnet, daß wichtige Qualitätsparameter des Holzstoffes nur im Labor oder erst am fertigen Produkt, z.B. dem fertigen Papier, erfaßt werden können. Da diese Laborwerte mit Zeitverzögerung gemessen werden, sind sie für eine Online-Regelung nicht unmittelbar nutzbar. Es kann eine zwischenzeitliche Fehlproduktion nicht ausgeschlossen werden. Um Fehlproduktionen möglichst auszuschließen, werden üblicherweise höherwertigere Rohstoffe eingesetzt als erforderlich, Rohstoffe stärker prozessiert als notwendig bzw. Hilfsstoffe, wie z. B. Leim, in größerem Umfang eingesetzt, als eigentlich zur Erzielung der gewünschten Qualität erforderlich wäre.

Beim Stand der Technik werden im wesentlichen folgende Strategien angewandt:
- Der Prozeß wird nach Erfahrungswerten gesteuert. Regeleingriffe sind dabei nur in geringem Umfang möglich. Die Produktqualität kann nur im nachhinein bestimmt werden. Eine große Streuung der Qualitätsparameter ist daher häufig nicht vermeidbar, insbesondere wenn die Qualität der eingesetzten Rohstoffe, d.h. Holzsorte, Qualität, Dicke, Feuchte und Alter, stark schwankt.
- Es wird versucht, den Prozeß über online meßbare Hilfsgrößen zu regeln. Regelbare Stellgrößen sind die Temperatur, Druck und Verweilzeit bei der Vorbehandlung der Ausgangsstoffe, mechanische Parameter am Refiner (Drehzahl und Spalt), das Verdünnungswasser und den Durchsatz am Refiner. Ein Zusammenhang zu den erwünschten Qualitätsparametern besteht jedoch nur mittelbar.

Es wurde auch bereits vorgeschlagen, speziell Infrarotspektroskopie zur Prozeßbeurteilung vorzusehen. Zum Einsatz der Infrarot-Spektroskopie bei der Holzstoffherstellung sind verschiedene Veröffentlichungen bekannt: In der DE 195 10 008 A1 wird ein Verfahren zur Prozeßführung bei der Zellstoff- und Papierherstellung vorbeschrieben, bei dem spektrale Kennwerte unterschiedlicher Wellenlängen zur Bestimmung der Ausgangsstoffe, und zur Bewertung der Rohstoffqualität herangezogen werden. Insbesondere über neuronale Netze können daraus Korrektursignale für die Regel- oder Steuereinrichtung verwendet werden. Als Beispiele für die Ausgangsstoffe bei der Zellstoff- und Papierherstellung sind dabei insbesondere Altpapier, Holz, Holzhackschnitzel und Holzfasern angegeben.

Weiterhin sind aus der WO 93/15389 A1 ein Verfahren und eine zugehörige Vorrichtung zur Bestimmung der sog. "Freeness", d.h. dem Mahlgrad von Refiner-Holzstoff, bekannt: Dabei werden spektrale Kennwerte von ausgewählten Wellenlängenbändern durch ein Rechenverfahren verarbeitet, um den gewünschten Qualitätsindex "Freeness" zu erhalten. Zur Kalibrierung werden gleichzeitig spektrale Messungen und Qualitätsmessungen durchgeführt, die Meßergebnisse werden als neue Variable verarbeitet und die Abhängigkeit der "Freeness" unter Verwendung der Hauptkomponenten-Methode bestimmt. Die bei diesem optischen Verfahren verwendeten Wellenlängen liegen dabei im Bereich zwischen 0,1 bis 10 µm.

Schließlich wird in der DE-A-37 12 096 ein Verfahren und eine Vorrichtung zur Herstellung von Papier aus einem fasrigen Papierstoff beschrieben, bei dem der Ausgangsstoff zunächst eine Refinereinheit durchläuft und anschließend zur Papiermaschine gelangt. Dabei wird mit einer Faser-Analysiereinheit der dem Refinervorgang ausgesetzte Papierstoff zum Erhalt charakteristischer Ausgangsinformationen analysiert. Diese Informationen werden mit Referenzinformationen verglichen, die bei der Herstellung von Papier gewünschter Qualität erhalten wurden.Im einzelnen werden in dieser Druckschrift Spektren mechanischer Größen erfaßt und daraus diesbezügliche Aussagen abgeleitet.

Untersuchungen zur Anwendung der Infrarot-Spektroskopie im Zusammenhang mit der Untersuchung von Holz werden in "Holzforschung und Holzverwertung" 40 (1988) 120, 41 (1989) 22 und 42 (1990) 25 beschrieben. Auf der Basis von IR-Spektren wird dort speziell der Anteil von Nadel- und Laubholz, von Rinde und Nadeln in Spangemischen ermittelt. In "Holzforschung" 47 (1993) 45 wird über die Bestimmung von phenolischen Hydroxylgruppen in gemahlenem Holzlignin mit der FTIR-Spektroskopie unter Verwendung der Verfahren der PCA- und der PLS-Analyse berichtet.

Davon ausgehend ist es Aufgabe der Erfindung, die komplette Prozeßführung bei der Herstellung von Holzstoff und insbesondere beim Betrieb von sog. Schleifern und Refinern weiter zu optimieren und eine zugehörige Vorrichtung zu schaffen.

Die Aufgabe ist erfindungsgemäß mit den Merkmalen des Patentanspruches 1 gelöst, wobei vorteilhafte Weiterbildungen in den abhängigen Ansprüchen angegeben sind. Die zugehörige Vorrichtung ist im einzigen Sachanspruch angegeben.

Gemäß dem erfindungsgemäßen Verfahren werden vorteilhafterweise direkt am im Schleifer oder Refiner erzeugten Holzstoff online spektroskopische Messungen durchgeführt. Die spektroskopischen Messungen erfolgen beispielsweise in Absorption, Emission, Lumineszenz im Spektralbereich von 0,1 µm bis 400 µm.

Die Spektren werden vorzugsweise mit unterschiedlichen Rechenverfahren ausgewertet und ermöglichen eine Vorhersage der erzielbaren Papierqualität. Auf der Basis dieser Vorhersage kann in den Prozeß regelnd eingegriffen werden, z.B. durch Veränderung der Rohstoffmischung, insbesondere durch Zumischung von Holz höherer/minderer Qualität, Veränderung der Parameter der Vorbehandlung, wie Temperatur, Druck und Dauer der Dampfbehandlung, Optimierung der Refinereinstellung wie Drehzahl, Spalt, Verdünnungswasser und Durchsatz. Damit ist vorteilhafterweise bei gleicher Produktqualität eine Ersparnis beim Rohstoffeinsatz eine Optimierung der Hilfsstoffe und des Energieverbrauches beim Refiner sowie eine Vermeidung von Fehlproduktionen durch minderwertige Qualität erreicht.

Das erfindungsgemäße Verfahren ist gegenüber dem abgehandelten Stand der Technik in den wesentlichen Punkten, wie dem Meßort, der Meßmethode, der Verarbeitung der Spektren der Modellbildung und der Prozeßführung, weiter entwickelt. Bei der zugehörigen Vorrichtung wird ein entsprechend programmierter Prozessor oder ein geeignetes Softwarepaket für einen herkömmlichen Digitalrechner bereitgestellt.

Die Messung erfolgt online unmittelbar nach dem Refiner oder Schleifer direkt im gemahlenen Stoff oder an einer mit Hilfe eines Probennehmers gewonnenen Stoffprobe. Die Stoffprobe wird je nach Aufgabenstellung aufbereitet, z.B. durch Aufkonzentrieren, Trocknen und/oder Bildung eines Probenblattes. An den Stoffproben können Labormessungen vorgenommen werden, um die Modelle für die Qualitätsparameter nachzutrainieren.

Wie erwähnt, wird bei der Erfindung mit elektromagnetischen Wellen im Bereich der Wellenlängen zwischen 100 nm und 400 µm, vorzugsweise im Bereich von 0,4 µm bis 100 µm, insbesondere 0,2 µm bis 10 µm, gearbeitet. Gemessen werden können neben Absorptions-, Emissions- , Lumineszenz- oder auch Raman-Spektren der Stoffproben. Speziell die Absorptionspektroskopie kann in Transmission, diffuser Reflexion oder gedämpfter Totalreflexion (ATR = attennated total reflection) erfolgen. Speziell die Anregung der Lumineszenz kann z.B. durch die Einstrahlung von elektromagnetischer Strahlung erfolgen (z.B. UV-Strahlung) oder durch eine spezifische chemische Reaktion (Chemolumineszenz) erfolgen.

Die Anregung der Emission kann z.B. durch Bestrahlung mit Elektronen erfolgen.

Bei Messung im Bereich des Infraroten (IR: 800 nm bis 20 µm) kann vorzugsweise die Fourier-Transformations-Spektroskopie (FTIR) eingesetzt werden. An inhomogen Stoffproben erfolgt die spektroskopische Messung mehrfach, d.h. durch mehrfache Probennahme oder an verschiedenen Meßorten der Stoffproben.

Die Spektren können durch folgende Maßnahmen vorverarbeitet werden:
- durch Fourier-Transformation
- Bei der Messung der Absorption durch diffuse Reflexion erfolgt eine Umrechnung in Kubelka-Munk-Einheiten und Korrektur von Mehrfachstreueffekten
- durch Normierung und Glättung der Spektren
- durch Ermittlung von für die Modellbildung ungeeigneten Spektren. Die Ausschaltung ungeeigneter Messungen kann z.B. durch Vergleich mit Referenzspektren erfolgen.
- Bei mehreren Spektren zu einer Probennahme durch Bildung von Mittelwerten.

Nach diesen ersten Verarbeitungsschritten werden aus Spektren ganz oder abschnittsweise folgende rechnerische Verfahren zur Ermittlung von Kenngrößen angewandt:
- Hauptkomponentenanalyse (PCA) und Beschreibung der Spektren durch die Hauptkomponenten
- Partial Least Square (PLS)-Verfahren
- Neuronale Netze
- Analytische Beschreibung eines Spektrums, z.B. im Bereich des IR durch Lage, Intensität und Breite der wichtigsten Absorptions- oder Emissionspeaks, Ermittlung dieser Größen z.B. mit einfachen Minimum-Maximum-Verfahren oder der 2. Ableitung.
Die Kenngrößen werden zur Modellierung der gewünschten Qualitätsparameter herangezogen.

Die Modelle für Berechnung der Qualitätsparameter können bei ausreichend großer Zahl von Daten vorzugsweise Neuronale Netze, Fuzzy-Systeme oder Multilineare Regressionsmodelle bzw. Kombinationen daraus sein. Alternativ zu rein datengetriebenen Modellen sind auch kombinierte Modelle möglich, bei denen analytisches Wissen eingebracht wird.

Die Aufstellung der Modelle, d.h. Auswahl der Modelleingangsgrößen und deren Validierung, erfolgt mit Labormessungen am Zwischen- und Endprodukt. Das Training der Modelle erfolgt online, z.B. jeweils nach jedem neuen Laborwert, wobei auch ein nur partielles Nachlernen möglich ist. Eine in der Spektrenvorverarbeitung integrierte Prüfung auf Modellgültigkeit ("Novelty Detection") entsprechend der älteren, nicht vorveröffentlichten DE 196 322 45 A1 kann im laufenden Prozeß rechtzeitig die Notwendigkeit einer neuen Trainingsphase anzeigen.

Weitere Einzelheiten und Vorteile der Erfindung ergeben sich aus der nachfolgenden Figurenbeschreibung von Ausführungsbeispielen anhand der Zeichnung in Verbindung mit den weiteren Patentansprüchen. Es zeigen
- Figur 1: ein Schema der Meßstellen bei der Herstellung von Holzstoff mit zwei Refinerstufen,
- Figur 2a und 2b: kontinuierliche Spektren von optischen Messungen an einer Faserstoffsuspension und von mechanischen Messungen an Fasern,
- Figur 3: ein Zustandsmodell für die Qualitätsparameter beim Holzstoff,
- Figur 4: ein Prozeßmodell für eine zweistufige Refineranlage,
- Figur 5: den schematischen Aufbau einer Prozeßoptimierung zum Steuern eines Refiners,
- Figur 6: eine Verdeutlichung der Vorverarbeitung und Verdichtung der Spektren,
- Figur 7: ein mehrstufiges Neuronales Netz und
- Figur 8: eine Vorrichtung zur optimierten Prozeßführung eines Schleifers oder Refiners.

Die Figuren werden nachfolgend teilweise gemeinsam beschrieben. Gleiche bzw. gleich wirkende Teile haben entsprechende Bezugszeichen.

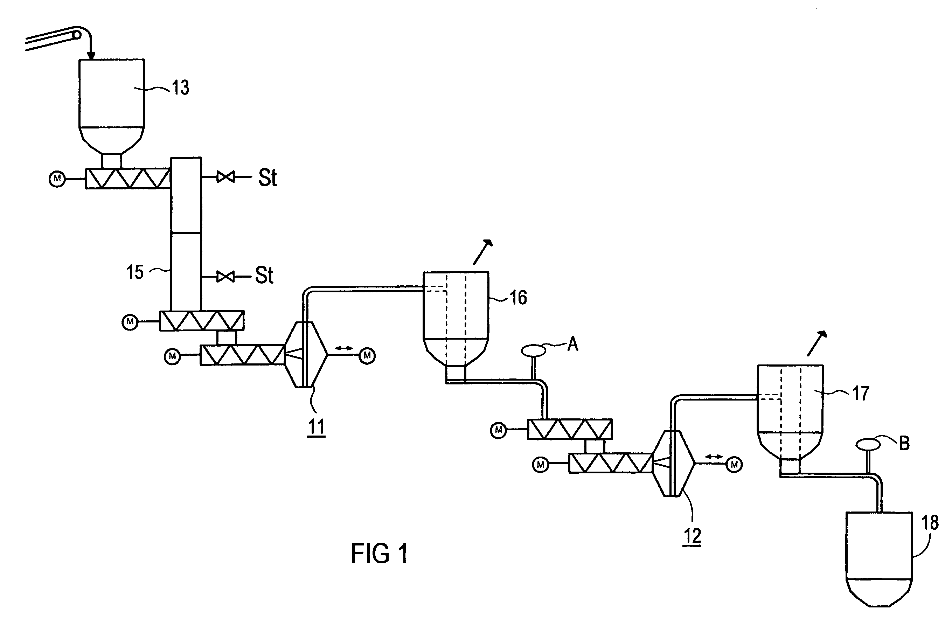

In Figur 1 ist eine zweistufige Refineranlage mit Refinerstufen 11 und 12 dargestellt. In einem Vorwärmer 13 werden Hackschnitzel bei Atmosphärendruck aufgeheizt und in einem Dämpfkessel 15 mit Druck und Temperatur behandelt. Über Förderschnecken werden die Hackschnitzel dem ersten Refiner 11 zugeführt. Im ersten Dampfabscheider 16 werden die Holzfasern vom Dampf getrennt, der zur Rückgewinnung mit Wärmeaustausch geht. Über Förderschnecken werden die Holzfasern einem zweiten Refiner 12 zugeführt. Im nachgeschalteten Dampfabscheider 17 werden die Holzfasern wiederum vom Dampf getrennt, der wiederum zur Rückgewinnung mit Wärmeaustausch geht. Die Holzfasern werden in einer sog. Latenzbütte 18 oder in einer Maschinenbütte gesammelt.

An signifikanten Stellen dieser Anordnung werden mittels geeigneter Spektrometer kontinuierliche Spektren im Infrarotbereich (IR: 1 bis 25 µm) gemessen. Beispielsweise wird in Figur 1 ein Spektrum A zwischen der Refinerstufe 11 und 12 und ein Spektrum B vor der Bütte 18 aufgenommen.

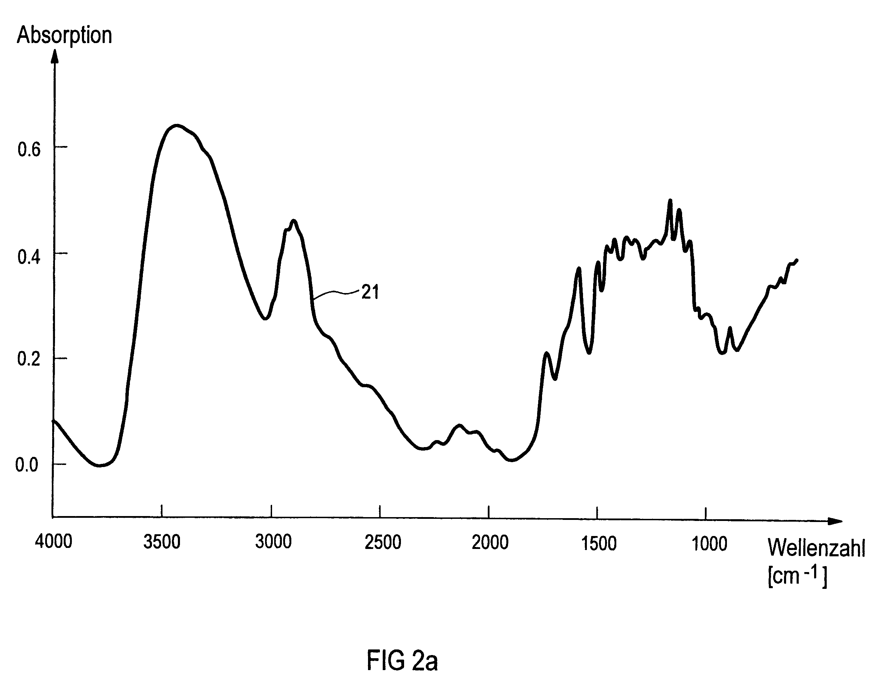

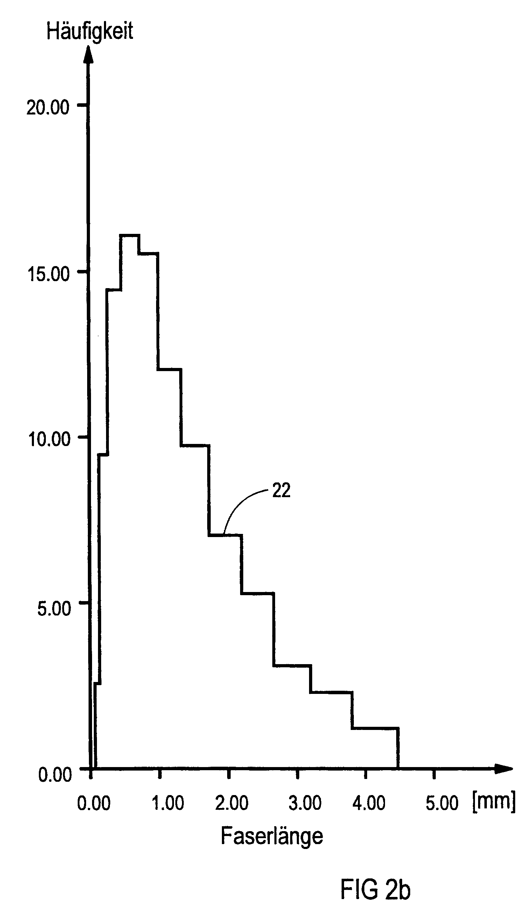

In Figur 2a kennzeichnet 21 beispielhaft den Verlauf eines MIR-Spektrums eines Refiner-Holzstoffes im Wellenzahlbereich von 4000 bis 750 cm⁻¹. In Figur 2b ist dagegen das Spektrum einer Faserlängenverteilung des Holzstoffes dargestellt, wie es sich aus einer Messung der mechanischen Eigenschaften ergibt. Die Grenzlinie 22 der Balkenverteilung gemäß Figur 2b gibt hier im wesentlichen einen Verlauf entsprechend einer sogenannten Bernoulli-Verteilung wieder. Die Verteilung der Siebfraktionen von einzelnen Fasern eines Faserstoffs hat einen ähnlichen Verlauf.

Die anhand der Figuren 2a bzw. 2b erhaltenen Spektren werden mittels unterschiedlichster mathematischer Methoden aufbereitet. Dabei geht es im wesentlichen um eine Vorverarbeitung der Spektren, um das Einbringen von analytischem Wissen, um eine mögliche Ausreißerkennung und bestimmungsgemäße Ableitung von Kenngrößen.

Die gemessenen Spektren können anschließend geglättet und normiert werden. Mit einem bekannten Minimum - Maximum Verfahren und der 2. Ableitung werden die Absorptionsmaxima als Peaks in den Spektren ermittelt und hinsichtlich Peaklage, Peakintensität, Peakbreite klassifiziert. Als brauchbar identifizierte Spektren können zu einer Mittelwertbildung herangezogen werden.

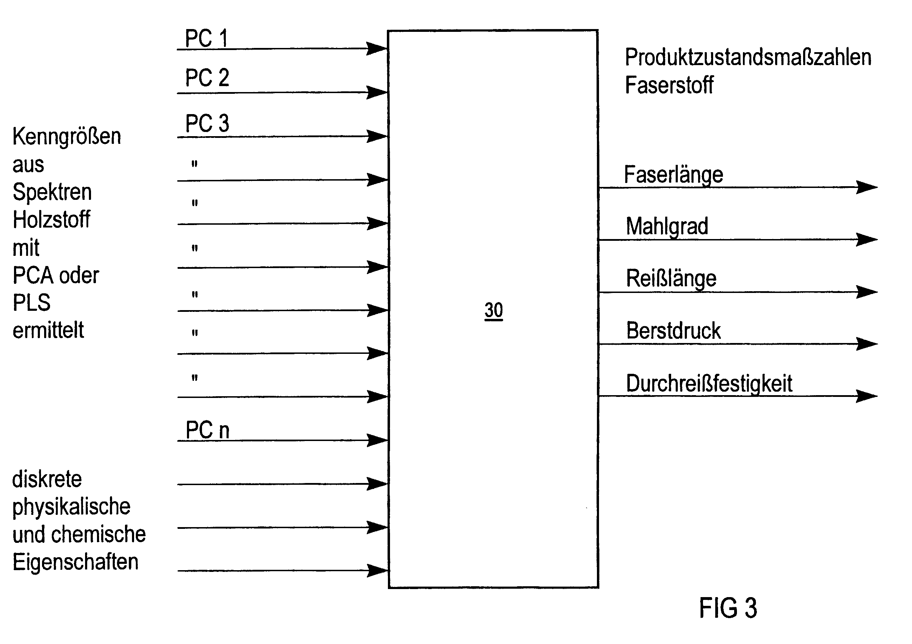

In Figur 3 bedeuten 30 ein Zustandsmodell des Refiners, aus dem im wesentlichen die Qualitätsparameter für den Holzstoff nach dem Refiner, wie z.B. Zugfestigkeit, Berstfestigkeit und Druckreißfestigkeit, berechnet werden. Dazu werden aus den Spektren entweder mit der sogenannten Hauptkomponentenanalyse (PCA = principal component analysis) oder mit der sogenannten PLS-Methode (partial least square) Kenngrößen PC1 bis PCn ermittelt und in das Zustandsmodell eingegeben. Zusätzlich werden in Figur 3 diskrete physikalische und chemische Eigenschaften, wie beispielsweise Temperatur oder Art bzw.Anteil von ggfs. verwendeten Behandlungschemikalien.Während der Bearbeitung. Erhalten werden Produktzustandsmaßzahlen für den erzeugten Faserstoff, wie insbesondere Faserlänge, Mahlgrad, Meßlänge, Berstdruck und Durchreißfestigkeit.

Bei der hier verwendeten Hauptkomponentenanalyse werden aus einer geeigneten Anzahl von Spektren, beispielsweise zwischen drei und zehn Spektren, sogenannte Scores zwecks Datenreduktion gebildet. Daraus wurden die Kenngrößen PC1 bis PCn, die Eingangsgrößen des Modells gemäß Figur 3 sind, berechnet. Mit einer zusätzlichen Eingabe von Produkt- bzw. Prozeßeigenschaften wird aus dem eigentlichen Zustandsmodell ein Prozeßmodell.

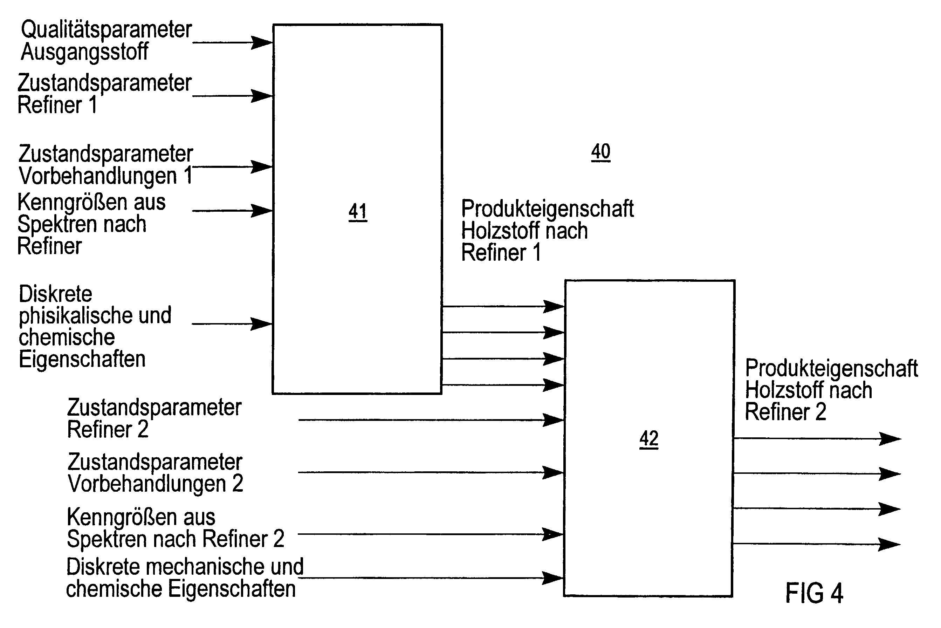

In Figur 4 ist speziell das Prozeßmodell für die Holzstoffherstellung mit 40, das aus zwei Teilmodellen für einen ersten Refiner und einen nachfolgenden zweiten Refiner besteht, bezeichnet: In Das Prozeßmodell für den ersten Refiner gehen den Kenngrößen aus den Spektren im wesentlichen die Qualitätsparameter des Ausgangsstoffes, wie beispielsweise Holzspezies, -qualität, -alter oder auch - Holzfeuchte, und die Zustandsparameter der ersten Refinersstufe ein. Dazu gehören die Betiebsparameter des Refiners selbst, wie RPM, Gap, Durchsatz, Leistungsaufnahme, Scheibendesign und -alter sowie Verdünnungswasser, aber auch die Zustandsparameter der notwendigen Vorbehandlungen des faserstoffes, wie insbesondere Druck, Temperatur, Zeit und Zusatzstoffe bei der Vorbehandlung. Neben den Kenngrößen PC1 bis Pcn können als als Eingangsgrößen für den Produktzustand vom Holzstoff nach Refiner 1 sowie die Zustandsparameter des Refiners 2 werden zusätzlich diskrete mechanische und chemische Eigenschaften verwendet werden.

Auf die diskreten physikalischen und/oder chemischen Eigenschaften kann zur Aufstellung des Zustands- und/oder Prozeßmodells ggfs. verzichtet werden, so daß die Modelle allein mit den aus den kontinuierlichen Spektren abgeleiteten Kenngrößen gebildet werden. Ausgangsgröße des zeistufigen Prozeßmodells sind in jedem Fall die Produkteigenschaften des Holzstoffes am Ende der Refinerlinie.

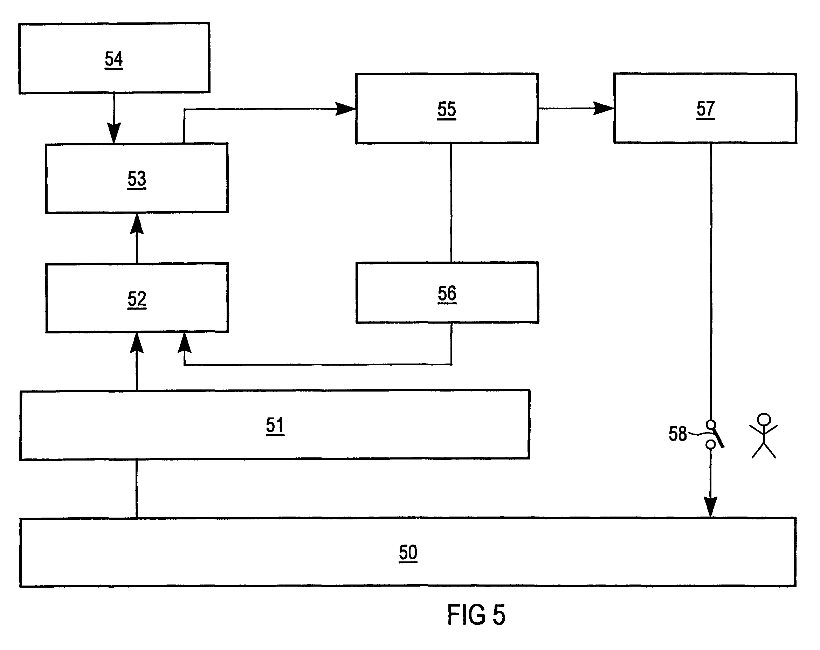

Die so erhaltenen Größen werden zur Prozeßführung eingesetzt. Dafür ist in Figur 5 der Prozeß allgemein mit 50 bezeichnet, woraus sich der aktuelle Prozeßzustand und die Spektren mit 51 ergibt. Das Prozeßmodell ist hier mit 52 bezeichnet, aus dem die Daten in eine Einheit zur Kostenfunktion 53 gegeben werden, die gleichzeitig mit Daten für Kosten und Preise aus der Einheit 54 beaufschlagt wird. Die Kostenfunktion 53 kann dabei fakultativ auf die Produktionskosten gerichtet sein oder eine Gewinnfunktion sein. Ein Optimierer 55 ermittelt daraus die Stellgrößen 56, die in das Prozeßmodell 52 zurückgekoppelt werden, und weiterhin die optimalen Stellgrößen 57 zur Prozeßführung der Refineranlage. Diese können über einen Schalter 58 vom Anlagenfahrer eingegeben werden, sofern sie als sinnvoll erkannt werden.

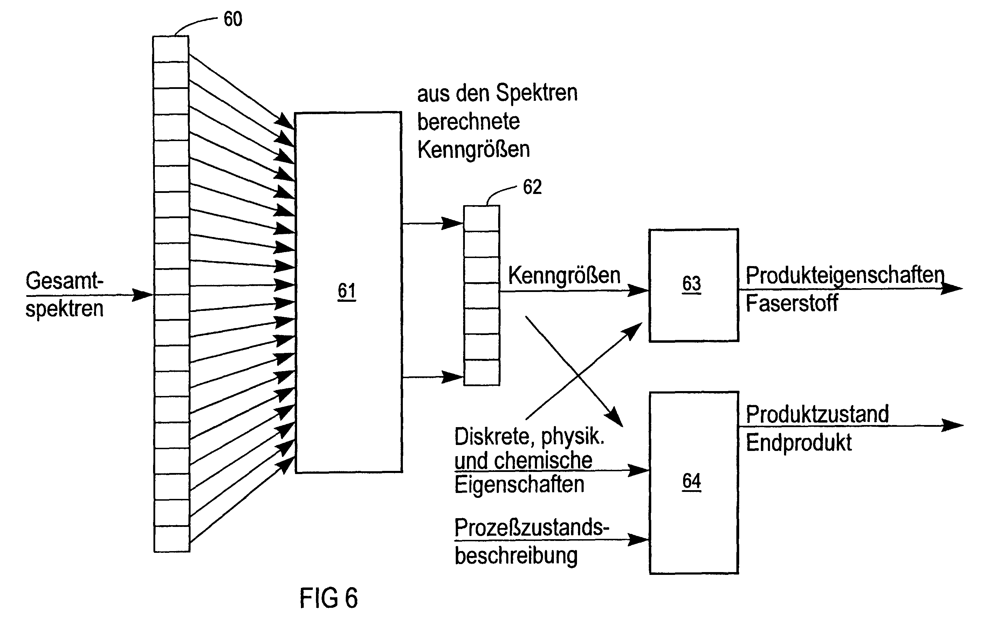

In Figur 6 ist dargestellt, daß eine Einheit 61 zur Vorverarbeitung und Verdichtung des Gesamtspektrums dient, aus dem entsprechend Einheit 62 die Kenngrößen berechnet werden. Die Kenngrößen fließen in das Zustandsmodell 63 und in das Prozeßmodell 64 ein, wobei in diesem Fall zusätzlich diskrete mechanische und/oder chemische Eigenschaften und die Prozeßzustandsbeschreibung das Zustandsmodell zum Prozeßmodell ergänzt. Vom Zustandsmodell 63 wird der Qualitätsparameter für den Faserstoff und aus dem Prozeßmodell 64 der Qualitätsparameter für das Endprodukt abgeleitet.

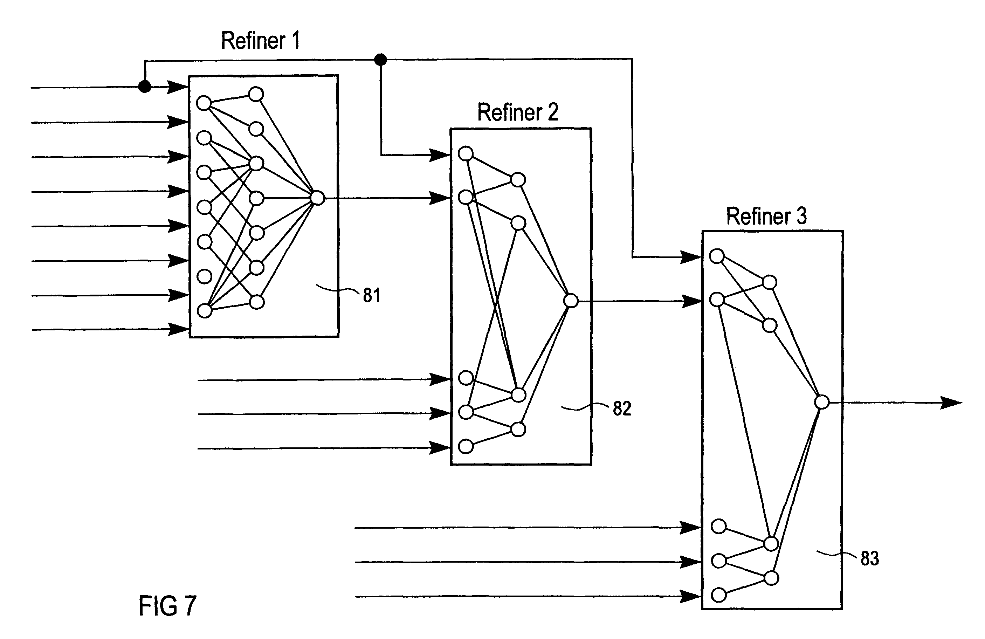

In Figur 7 ist die Anwendung von neuronalen Netzen für die Modellierung verdeutlicht. Ein Gesamtmodell 80 für eine Refinerlinie besteht hier aus drei Teilmodellen 81, 82 und 83 für je eine Refinerstufe, für die also je ein separates neuronales Netz eingesetzt wird. Dabei werden - entsprechend der Struktur der gesamten Anlage - die Ausgänge des ersten Teilnetzes 81 zusammen mit weiteren Eingangsgrößen als Eingänge für das zweite Teilnetz 82 und dessen Ausgänge zusammen mit weiteren Eingangsgrößen als Eingänge für das dritte Teilnetz 83 verwendet.

Bei der Aufstellung der Modelle gemäß den Figuren 3 bis 7 kann von neuronalen Netzen und/oder Fuzzy-Logik Gebrauch gemacht werden. Insbesondere geht es darum, die Gültigkeit der Modelle zu validieren, was durch ein online-Training der einzelnen Modelle bzw. der Teilmodelle erfolgen kann. Dabei kann es für die Praxis wichtig sein, durch rechnergestützte Auswahl aller informationstragender Daten eine Überprüfung der jeweils erhaltenen Ergebnisse vorzunehmen. Dieses Verfahren wurde als sog. "Novelty Detection" vorgeschlagen und ermöglicht, neue Datensätze in das Auswerteverfahren einzubringen. Bei Vorliegen nicht konsistenter Ergebnisse ist ein Nachtrainieren der Modelle notwendig.

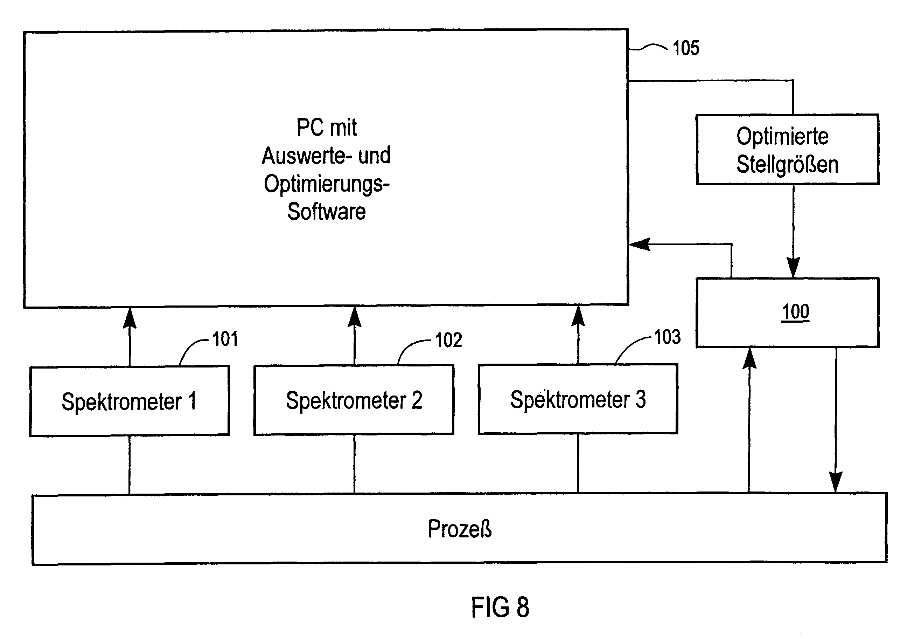

Figur 8 zeigt, wie eine Auswerte- und Optimierungssoftware anhand eines Rechners 105 mit in die gesamte Prozeßführung eingebunden wird. Dabei werden optimierte Stellgrößen erzeugt, die ein bekanntes Automatisierungsgerät als Prozeßleitsystem 100 beaufschlagen, das in sich bekannter Weise mit der eigentlichen Anlage zur Durchführung des Prozesses in Wechselwirkung steht. In der Praxis wird die herkömmliche Anlage lediglich durch mehrere Spektrometer und ein zugehöriges Software-Paket, das auf üblichen Rechnern abläuft, ergänzt.

## Patentansprüche

1. Verfahren zur Prozeßführung und zur Prozeßoptimierung beim Herstellen von Faserstoff aus Holz, insbesondere von Holzschliff und/oder Refinerstoff, durch Mahlen und/oder Schleifen des Holzes und Überführen in eine Holzsuspension unter Einsatz wenigstens eines Zustandsmodells und/oder Prozeßmodells, mit folgenden Merkmalen:
a) an mindestens einer Stelle werden am Faserstoff oder an der Stoffsuspension oder einem aus der Stoffsuspension gewonnenen Probenblatt kontinuierliche Spektren von elektromagnetischer Strahlung und/oder kontinuierliche Spektren von mechanischen Eigenschaften gemessen,
b) durch mathematische Auswertung der kontinuierlichen Spektren werden Kenngrößen (PCl,...,PCn) für den Faserstoff und/oder die Holzsuspension ermittelt,
c) die Kenngrößen (PCl,...,PCn) und Labormessungen der Produkteigenschaften werden in das Zustandsmodell und/oder es werden zusätzlich Prozeßeigenschaften in das Prozeßmodell eingegeben, wodurch die Modelle verifiziert werden,
d) mit Hilfe des so aufgestellten Zustands- und/oder Prozeßmodells werden optimierte Stellgrößen für ein vorhandenes Prozeßleitsystem (100) gebildet.

2. Verfahren nach Anspruch 1, **dadurch gekennzeichnet, daß** an mindestens einer Stelle am Faserstoff oder einem aus der Stoffsuspension gewonnenen Probenblatt und/oder am Zellstoff diskrete physikalische und/oder chemische Eigenschaften erfaßt werden.

3. Verfahren nach Anspruch 1 und Anspruch 2, **dadurch gekennzeichnet, daß** die diskreten physikalischen und/oder chemischen Eigenschaften zur Aufstellung des Zustands- und ggfs. des Prozeßmodells zusätzlich verwendet werden.

4. Verfahren nach Anspruch 1, **dadurch gekennzeichnet, daß** bei Wellenlänge der elektromagnetischen Strahlung zwischen 100 nm und 400 µm gemessen wird.

5. Verfahren nach Anspruch 4, **dadurch gekennzeichnet, daß** die elektromagnetische Strahlung in Absorptions-, Emissions-, Lumineszenz- oder als Raman-Spektrum erfaßt wird.

6. Verfahren nach Anspruch 4, **dadurch gekennzeichnet, daß** die elektromagnetische Strahlung in Transmission, direkter oder diffuser Reflexion oder gedämpfter Totalreflexion (ATR) erfaßt wird.

7. Verfahren nach Anspruch 1, **dadurch gekennzeichnet, daß** als kontinuierliche Spektren der mechanischen Eigenschaften die Faserlängenverteilung oder die Siebfraktionen der Fasern herangezogen werden.

8. Verfahren nach Anspruch 2, **dadurch gekennzeichnet, daß** als diskrete physikalische und/oder chemische Eigenschaften des Stoffes die Feststoffkonzentration Konsistenz, die Temperatur und/oder die Durchflüsse der Stoffsuspension erfaßt werden

9. Verfahren nach Anspruch 1, **dadurch gekennzeichnet, daß** an einer vorgegebenen Anzahl von Spektren eine Hauptkomponentenanalyse durchgeführt und zur Datenreduktion eine entsprechende Anzahl von Scores ausgewählt wird und daß daraus die Kenngrößen für die Modellbildung ermittelt werden.

10. Verfahren nach Anspruch 9, **dadurch gekennzeichnet, daß** die Spektren vorverarbeitet und verdichtet werden, und daß die spezifischen Kennwerte der Spektren, insbesondere die Hauptkomponenten, zur Beschreibung des Produktzustandes ausgewählt werden und unmittelbar in das Prozeßmodell eingegeben werden.

11. Verfahren nach Anspruch 9, **dadurch gekennzeichnet, daß** die Spektren vorverarbeitet und verdichtet werden, daß deren spezifische Kenngrößen, insbesondere die Hauptkomponenten, in das Zustandsmodell eingebracht werden, und daß am Ausgang des Zustandsmodells die Produkteigenschaften gebildet und unmittelbar in das Prozeßmodell eingegeben werden.

12. Verfahren nach Anspruch 1, **dadurch gekennzeichnet, daß** für die Modellbildung ungeeignete Spektren durch Plausibilitätsprüfung eliminiert werden.

13. Verfahren nach Anspruch 1 oder Anspruch 2, **dadurch gekennzeichnet, daß** die Messung der kontinuierlichen Spektren zwischen den Refinerstufen erfolgt.

14. Verfahren nach Anspruch 1, **dadurch gekennzeichnet, daß** die durch Auswertung der Spektren erhaltenen Kenngrößen zur Steuerung und/oder Regelung der Refinerstufen herangezogen werden, wobei die Aufbereitungsstufen einzeln oder auch im Verbund optimiert werden.

15. Verfahren nach Anspruch 13, **dadurch gekennzeichnet, daß** zur Steuerung und/oder Regelung der Refinerstufen die Qualitätsparameter des aufbereiteten Holzstoffes, wie spezifische Mahlarbeit, der Mahlspalt, der Dampf- und Wassereinsatz modelliert werden.

16. Verfahren nach Anspruch 15, **dadurch gekennzeichnet, daß** die Modellansätze neben der Vorhersage der Produktqualität auch zur Berechnung des Dampf- und/oder Wassereinsatzes und/oder Durchsatz für die Refiner herangezogen werden.

17. Verfahren nach Anspruch 15, **dadurch gekennzeichnet, daß** der Modellansatz mit den Qualitätsparametern zur Prozeßoptimierung eingesetzt wird.

18. Verfahren nach einem der vorhergehenden Ansprüche, **dadurch gekennzeichnet, daß** eine Kostenfunktion gebildet wird, die mit einem Optimierer durch geeignete Variation der Stellgrößen optimiert wird.

19. Verfahren nach Anspruch 17, **dadurch gekennzeichnet, daß** die Optimierung durch Fuzzylogik, Neuronale Netze und/oder genetische Algorithmen erfolgt.

20. Verfahren nach Anspruch 18, **dadurch gekennzeichnet, daß** die Kostenfunktion eine Kostenfunktion für die Produktionskosten und/oder eine Gewinnfunktion ist.

21. Verfahren nach einem der vorhergehenden Ansprüche, **dadurch gekennzeichnet, daß** das Modell und/oder die Teilmodelle online trainiert werden.

22. Verfahren nach einem der vorhergehenden Ansprüche, **dadurch gekennzeichnet, daß** durch rechnergestützte Auswahl aller informationstragenden Daten eine Überprüfung von erhaltenen Ergebnissen durchgeführt wird( "Novelty Detection").

23. Verfahren nach Anspruch 22, **dadurch gekennzeichnet, daß** bei Vorliegen nichtkonsistenter Ergebnisse ein Nachtrainieren erfolgt.

24. Vorrichtung zur Durchführung des Verfahrens nach einem der Ansprüche 1 bis 23, bestehend aus wenigstens einem Spektrometer (101, 102, 103) zur Messung von kontinuierlichen Spektren, aus einem einen Digitalrechner (105) zur mathematischen Auswertung(PC1 ...PCn) der kontinuierlichen Spektren zwecks Ermittlung der Kenngrößen (PC1 ... PCn) und zur Aufstellung des Zustandsmodells und/oder Prozeßmodells aus den Kenngrößen (PC1 ...PCn), gegebenenfalls den diskreten physikalischen und/oder chemischen Eigenschaften als Prozeßmeßgrößen, sowie aus einem Prozeßleitsystem (100) zur Prozeßoptimierung beim Herstellen von Faserstoff aus Holz unter Verwendung der optimierten Stellgrößen.

## Claims

1. Method for process management and for process optimisation in the production of fibre pulp from wood, in particular ground-wood and/or refiner pulp, by milling and/or grinding the wood and transfer to a wood suspension while using at least one state model and/or process model, having the following features:
a) at at least one point, continuous spectra of electromagnetic radiation and/or continuous spectra of mechanical properties are measured from the fibre pulp or from the pulp suspension or from a sample sheet obtained from the pulp suspension,
b) characteristic quantities (PC1, ..., PCn) for the fibre pulp and/or the wood suspension are determined by mathematical evaluation of the continuous spectra,
c) the characteristic quantities (PC1, ..., PCn) and laboratory measurements of the product properties are entered into the state model and/or process properties are additionally entered into the process model, by means of which the models are verified,
d) optimised manipulated variables for an existing process control system (100) are formed with the aid of the state and/or process model set up in this way.

2. Method according to Claim 1, **characterised in that**, at at least one point, discrete physical and/or chemical properties are recorded from the fibre pulp or from a sample sheet obtained from the pulp suspension and/or from the chemical pulp.

3. Method according to Claim 1 and Claim 2, **characterised in that** the discrete physical and/or chemical properties are additionally used for setting up the state model and optionally the process model.

4. Method according to Claim 1, **characterised in that** measurements are taken at a wavelength of the electromagnetic radiation of between 100 nm and 400 µm.

5. Method according to Claim 4, **characterised in that** the electromagnetic radiation is recorded in an absorption, emission or luminescence spectrum or as a Raman spectrum.

6. Method according to Claim 4, **characterised in that** the electromagnetic radiation is recorded in transmission, direct or diffuse reflection or attenuated total reflection (ATR).

7. Method according to Claim 1, **characterised in that** the fibre length distribution or the screen fractions of the fibres are employed as continuous spectra of the mechanical properties.

8. Method according to Claim 2, **characterised in that** the fibre pulp concentration, consistency, the temperature and/or the flow rates of the pulp suspension are employed as discrete physical and/or chemical properties of the pulp.

9. Method according to Claim 1, **characterised in that** a principal component analysis is carried out on a predetermined number of spectra and, for data reduction, a corresponding number of scores is selected, and **in that** the characteristic quantities for the model formation are determined therefrom.

10. Method according to Claim 9, **characterised in that** the spectra are pre-processed and compressed, and **in that** the specific characteristic quantities of the spectra, in particular the principal components, are selected for describing the product state and are entered directly into the state model.

11. Method according to Claim 9, **characterised in that** the spectra are pre-processed and compressed, **in that** their specific characteristic quantities, in particular the principal components, are introduced into the state model, and **in that** the product properties are formed at the output of the state model and are entered directly into the process model.

12. Method according to Claim 1, **characterised in that** spectra unsuitable for the model formation are eliminated by plausibility testing.

13. Method according to Claim 1 or Claim 2, **characterised in that** the measurement of the continuous spectra is carried out between the refiner stages.

14. Method according to Claim 1, **characterised in that** the characteristic quantities obtained by evaluating the spectra are employed for controlling and/or regulating the refiner stages, the preparation stages being optimised individually or in combination.

15. Method according to Claim 13, **characterised in that** the quality parameters of the prepared wood pulp, such as specific milling energy, milling gap, the steam and water utilisation are modelled in order to control and/or regulate the refiner stages.

16. Method according to Claim 15, **characterised in that** the model approaches are also used for calculating the steam and/or water utilisation and/or the throughput for the refiners, besides predicting the product quality.

17. Method according to Claim 15, **characterised in that** the model approach is used with the quality parameters for the process optimisation.

18. Method according to one of the preceding claims, **characterised in that** a cost function is formed and is optimised by an optimiser through suitable variation of the manipulated variables.

19. Method according to Claim 17, **characterised in that** the optimisation is carried out using fuzzy logic, neural networks and/or genetic algorithms.

20. Method according to Claim 18, **characterised in that** the cost function is a cost function for the production costs and/or a profit function.

21. Method according to one of the preceding claims, **characterised in that** the model and/or the submodels are trained online.

22. Method according to one of the preceding claims, **characterised in that** a check of the results obtained ("novelty detection") is carried out by computer-assisted selection of all data carrying information.

23. Method according to Claim 22, **characterised in that** retraining is carried out when there are inconsistent results.

24. Device for carrying out the method according to one of Claims 1 to 23, consisting of at least one spectrometer (101, 102, 103) for measuring continuous spectra, a digital computer (105) for mathematical evaluation (PC1 ...PCn) of the continuous spectra with a view to determining the characteristic quantities (PC1 ... PCn) and for setting up the state model and/or process model from the characteristic quantities (PC1 ...PCn), optionally the discrete physical and/or chemical properties as process measured variables, as well as a process control system (100) for process optimisation in the production of fibre pulp from wood while using the optimised manipulated variables.

## Revendications

1. Procédé pour conduire un processus et pour optimiser un processus lors de la fabrication de matières fibreuses en bois, notamment de la pâte mécanique et/ou de la pâte de raffineur, par broyage et/ou défibrage du bois et transformation en une suspension de bois en utilisant au moins un modèle d'état et/ou un modèle de processus ayant les caractéristiques suivantes :
a) on mesure en au moins un point sur la matière fibreuse ou sur la suspension de matière ou sur une feuille d'échantillon obtenue à partir de la suspension de matière des spectres continus de rayonnement électromagnétique et/ou des spectres continus de propriétés mécaniques,
b) on forme, en exploitant mathématiquement les spectres continus, des grandeurs caractéristiques (PCl, ..., PCn) pour la suspension de bois et/ou pour la matière fibreuse,
c) on entre les grandeurs caractéristiques (PCl, ..., PCn) et des mesures de laboratoire des propriétés associées au produit dans le modèle d'état et/ou on entre en plus des propriétés du processus dans le modèle de processus de manière à vérifier les modèles,
d) à l'aide du modèle d'état et/ou de processus ainsi formé, on forme des grandeurs réglantes optimisées pour un système (100) existant de conduite de processus.

2. Procédé suivant la revendication 1, **caractérisé en ce que** l'on détecte en au moins un point sur la matière fibreuse ou sur une feuille échantillon obtenue à partir de la suspension de matière et/ou sur la pâte cellulosique des propriétés physiques et/ou des propriétés chimiques discrètes.

3. Procédé suivant la revendication 1 et la revendication 2, **caractérisé en ce que** l'on utilise les propriétés physiques et/ou chimiques discrètes pour établir le modèle d'état et le cas échéant le modèle de processus.

4. Procédé suivant la revendication 1, **caractérisé en ce que** l'on mesure à des longueurs d'onde du rayonnement électromagnétique comprises entre 100 nm et 400 µm.

5. Procédé suivant la revendication 4, **caractérisé en ce que** l'on détecte le rayonnement électromagnétique en tant que spectre d'absorption, d'émission, de luminescence ou de spectre Raman.

6. Procédé suivant la revendication 4, **caractérisé en ce que** l'on détecte le rayonnement électromagnétique en transmission, en réflexion directe ou diffuse, ou en réflexion totale atténuée (ATR).

7. Procédé suivant la revendication 1, **caractérisé en ce que** l'on utilise comme spectres continus des propriétés mécaniques la répartition de longueur des fibres ou les fractions granulométriques des fibres.

8. Procédé suivant la revendication 2, **caractérisé en ce que** l'on utilise comme propriétés physiques et/ou chimiques discrètes la concentration en matière solide, la consistance, le débit et/ou la température de la suspension de matière.

9. Procédé suivant la revendication 1, **caractérisé en ce que** l'on effectue sur un nombre donné à l'avance de spectre une analyse de composantes principales et pour la réduction des données on choisit un nombre adéquat de scores et on en détermine les grandeurs caractéristiques pour la formation du modèle.

10. Procédé suivant la revendication 9, **caractérisé en ce que** l'on prétraite les spectres et on les comprime et **en ce que** l'on sélectionne les valeurs caractéristiques spécifiques des spectres, notamment les composantes principales pour décrire l'état du produit et on les entre directement dans le modèle d'état.

11. Procédé suivant la revendication 9, **caractérisé en ce que** l'on prétraite et comprime les spectres **en ce que** l'on introduit les valeurs caractéristiques spécifiques des spectres, notamment les composantes principales, dans le modèle d'état, et **en ce que** l'on forme à la sortie du modèle d'état les propriétés du produit et on les introduit directement dans le modèle de processus.

12. Procédé suivant la revendication 9, **caractérisé en ce que** pour la formation du modèle, on élimine les spectres qui ne conviennent pas par vérification de plausibilité.

13. Procédé suivant la revendication 1 ou 2, **caractérisé en ce que** l'on effectue la mesure des spectres continus entre les étages de raffinage.

14. Procédé suivant la revendication 1, **caractérisé en ce que** l'on utilise les grandeurs caractéristiques obtenues par l'exploitation des spectres pour commander et/ou pour réguler les étages de raffinage, les étages de préparation étant optimisés individuellement ou également en combinaison.

15. Procédé suivant la revendication 1, **caractérisé en ce que** pour commander et/ou réguler les étages de raffinage, on modélise les paramètres de qualité de la pâte mécanique préparée comme notamment le travail spécifique de raffinage, l'intervalle de broyage, l'apport de vapeur et d'eau.

16. Procédé suivant la revendication 15, **caractérisé en ce que** l'on utilise les équations du modèle, outre pour prédire la qualité du produit, également pour calculer les apports de vapeur et/ou d'eau et/ou le débit pour le raffinage.

17. Procédé suivant la revendication 15, **caractérisé en ce que** l'on utilise l'équation du modèle ayant les paramètres de qualité pour l'optimisation du procédé.

18. Procédé suivant l'une des revendications précédentes, **caractérisé en ce que** l'on forme une fonction de coût que l'on optimise avec un optimiseur en faisant varier d'une matière appropriée les grandeurs réglantes.

19. Procédé suivant la revendication 17, **caractérisé en ce que** l'on effectue l'optimisation par logique floue, par des réseaux neuronaux et/ou par des algorithmes génétiques.

20. Procédé suivant la revendication 18, **caractérisé en ce que** l'on utilise comme fonction de coût une fonction de coût pour les coûts de production et/ou une fonction de profit.

21. Procédé suivant l'une des revendications précédentes, **caractérisé en ce que** l'on fait subir au modèle et/ou aux sous-modèles un apprentissage on line.

22. Procédé suivant l'une des revendications précédentes, **caractérisé en ce qu'**il s'effectue par une sélection assistée par ordinateur de toutes les données porteuses d'informations un contrôle des résultats obtenus ("Novelty Detection").

23. Procédé suivant la revendication 22, **caractérisé en ce qu'**il s'effectue un post-apprentissage en présence de résultats non consistants.

24. Dispositif pour la mise en oeuvre du procédé suivant l'une des revendications 1 à 23, constitué d'au moins un spectromètre (101, 102, 103) de mesure de spectres continus, d'un ordinateur (105) numérique d'exploitation (PCl, ..., Pcn) mathématique des spectres continus en vue de déterminer les grandeurs (PCl, ..., Pcn) caractéristiques et d'établir le modèle d'état et/ou le modèle de processus à partir des grandeurs caractéristiques (PCl, ..., Pcn) et, le cas échéant, les propriétés discrètes physiques et/ou chimiques en tant que grandeurs de processus, ainsi que d'un système (100) de conduite de processus destiné à optimiser le processus lors de la fabrication de matière fibreuse en bois en utilisant les grandeurs réglantes optimisées.
